# EUROPÄISCHE PATENTANMELDUNG

(11) **EP 4 578 533 A2**
(43) Veröffentlichungstag der Anmeldung: **02.07.2025**
(21) Anmeldenummer: 25171750.0
(22) Anmeldetag: 20.09.2018
(51) Int. Cl.: B01D 63/02

(54) **GAS-AUSTAUSCH-EINHEIT**

(30) Priorität: 20.09.2017 DE 102017008781; 29.03.2018 LU 100759
(62) Teilanmeldung aus: 18785514.3
(71) Anmelder: Hemovent GmbH, 52076 Aachen (DE)
(72) Erfinder: MARSEILLE, Oliver, 52066 Aachen (DE); NOBIS, Andreas, 52146 Würselen (DE); NÖTZEL, Stefan, 52064 Aachen (DE); HESSELMANN, Felix, 52066 Aachen (DE)
(74) Vertreter: Sattler de Sousa e Brito, Clara

(57) **Zusammenfassung**

Gas-Austausch-Einheit umfassend einen Einlass, der azentrisch schräggestellt ist und ein Verfahren zur Herstellung einer Gas-Austausch-Einheit.

## Beschreibung

Die Erfindung betrifft eine Gas-Austausch-Einheit und ein Verfahren zum Herstellen einer Gas-Austausch-Einheit.

### HINTERGRUND DER ERFINDUNG

Bei Patienten mit lebensbedrohlicher Lungenerkrankung kann die Lungenfunktion über eine künstliche Lunge (Oxygenator oder Gas-Austausch-Einheit) aufrechterhalten werden, bis sich die Lunge erholt und sich die natürliche Lungenfunktion wiedereinstellt. Hierbei wird durch einen extrakorporalen Kreislauf das Blut dem Patienten entnommen und nach Behandlung im Oxygenator wieder zurückgeführt.

Die meisten bekannten Oxygenatoren wurden im Rahmen der Entwicklung der Herz-Lungen-Maschine zur Benutzung während herzchirurgischer Eingriffe von wenigen Stunden entwickelt und sind meist nicht für eine längerfristige Anwendung optimiert. Hingegen ist die Anwendungsdauer im Rahmen einer Lungenunterstützung oft deutlich länger und kann einige Wochen andauern. Während einer langen Anwendungsdauer eines bekannten Oxygenators kann es beispielsweise zur Hämolyse und Thrombenbildung durch eine suboptimale Strömungsführung mit hohen Strömungswiderständen und schlecht ausgewaschenen Bereichen kommen. Auch nimmt die Austauschleistung solcher Oxygenatoren ab, so dass diese während der Therapie ersetzt werden müssen, wobei es zu Komplikationen und auch Blutverlust bzw. starker Blutverdünnung kommen kann.

Übliche Oxygenatoren bestehen zum Beispiel aus gestapelten Hohlfasermatten, die wechselseitig rechtwinklig zueinander angeordnet sind. Im Randbereich werden die Matten durch eine Vergussmasse mittels eines Schleuder- oder Zentrifugalverfahren 4-fach eingebettet, wodurch sich eine quadratische Querschnittsfläche ergibt, durch die das Blut fließt. Um das Blut möglichst gleichmäßig über die Faserfläche zu verteilen, werden bei den üblichen Oxygenatoren gelochte Verteilerplatten verwendet, die im Einlassbereich und Auslassbereich des Oxygenators eingebaut werden. Ein derartiger Oxygenator ist beispielsweise aus der WO 2017/211460 A1.

### ZUSAMMENFASSUNG DER ERFINDUNG

Es ist Aufgabe der vorliegenden Erfindung, die Strömungsführung in einem Oxygenator zu verbessern, so dass die Bedürfnisse der mittel- bis langfristigen Lungenunterstützung in einfacher Art und Weise erfüllt werden können.

Die erfindungsgemäße Lösung der Aufgabe erfolgt durch die Merkmale der unabhängigen Ansprüche. Weiterbildungen der Erfindung ergeben sich aus den Unteransprüchen.

Erfindungsgemäß wird eine Gas-Austausch-Einheit angegeben, welche Hohlfasermatten umfasst, wobei die Gas-Austausch-Einheit einen Einlass umfasst, der azentrisch schräggestellt ist. Um die Blutströmung durch die Gas-Austausch-Einheit möglichst homogen über die kreisförmige Faserfläche zu verteilen und hier insbesondere den Randbereich hinreichend zu durchströmen wird das Blut durch den azentrisch schräggestellten Einlass mit einer Rotation versehen. Die Hohlfasermatten weisen eine kreisförmige Querschnittsfläche auf. Der Einlass der Gas-Austausch-Einheit ist in einer Weiterbildung zu den Faserlagen der Hohlfasermatten azentrisch angeordnet.

Wie folgend beschrieben, wird durch die erfindungsgemäßen Maßnahmen eine Gas-Austausch-Einheit gebildet, bei der auf die Verteilerplatten verzichtet und die Strömung des Fluids (insbesondere des Blutes) mit anderen Mitteln oder Maßnahmen gleichmäßig über die Faserfläche gebildet aus den Hohlfasermatten verteilt. Die erfindungsgemäße Gas-Austauch-Einheit hat eine höhere Anwendungsdauer oder Standzeit als bekannte Oxygenatoren. Zudem ist die Wahrscheinlichkeit einer Blutschädigung bei der angegebenen Gas-Austausch-Einheit gering.

Die Hohlfasermatten der Gas-Austausch-Einheit sind aus Hohlfasern gebildet. Die Gas-Austausch-Einheit kann eine oder mehrere Hohlfasermatten umfassen. Der Faserverlauf einer Fasermatte zu dem Faserverlauf einer weiteren Fasermatte kann in einem Winkel angeordnet sein. Durch die Kreuzung der Fasermatten kann die Gastaustauschcharakteristik als auch die Blutführung verbessert werden.

Die Gas-Austausch-Einheit wird auch als Oxygenator bezeichnet. Ein Oxygenator ist ein Gerät, bei dem Blut mit Sauerstoff angereichert und Kohlenstoffdioxid aus dem Blut entfernt werden kann. Somit kann mittels des Oxygenators sowohl kurzfristig als auch über längere Zeiträume hinweg die Lunge ersetzt oder unterstützt werden.

Die Gas-Austausch-Einheit sieht in einer Weiterentwicklung vor, dass der Einlass stirnseitig an einem Gehäuse der Gas-Austausch-Einheit angeordnet ist. Die Anordnung des Einlasses an einer Stirnseite der Gas-Austausch-Einheit ermöglicht eine homogene Durchströmung der Hohlfasermatten. Das Gehäuse der Gas-Austausch-Einheit weist ein Einlassgehäuse auf.

Gemäß einer Weiterentwicklung der Gas-Austausch-Einheit, ist vorgesehen, dass der Einlass mit dem Einlassgehäuse verbunden ist, dessen Oberfläche Strukturen aufweist. Geeignete Strukturen auf der inneren blutführenden Oberfläche des Einlassgehäuses verteilen die Strömung zusätzlich möglichst homogen. Dabei ist der Einlass schräggestellt, wenn ein Winkel zwischen einer Mittelachse des Einlasses und einer Ebene, die das Einlassgehäuse aufweist, größer als 0° und/oder kleiner als 90° ist.

In einer Weiterentwicklung der Gas-Austausch-Einheit ist vorgesehen, dass die Strukturen schaufelförmig, stegförmig oder als Querstreben auf der Oberfläche des Einlassgehäuses angeordnet sind. Durch die Strukturen, die schaufelförmig, stegförmig oder als Querstreben angeordnet sind, kann die Durchströmung oder Beschickung der Faserflächen in der Gas-Austausch-Einheit gleichmäßig verteilt erfolgen. Die Durchströmung erfolgt gleichmäßig sowohl im inneren als auch im äußeren Bereich der Hohlfasermatten.

Gemäß einer Weiterentwicklung der Gas-Austausch-Einheit, ist vorgesehen, dass der Einlass eine Querschnittsfläche aufweist, die sich in Strömungsrichtung kontinuierlich aufweitet, so dass die Strömungsgeschwindigkeit langsam und nicht stoßartig reduziert wird. Das Verhältnis zwischen Durchmesser (dD) und Lauflänge (dL) des Einlasses von dD/2dL<1 ist vorteilhaft. (Mit anderen Worten das Verhältnis zwischen Radius des Einlasses und Lauflänge ist kleiner als 1). Dieses Verhältnis ändert sich über die Lauflänge. Es kann sich auch im Bereich des Einlasses graduell ändern.

Gemäß einer Weiterbildung, ist eine Durchmessererweiterung des Einlasses über die Lauflänge des Einlasses kleiner als 45°.

Gemäß einer Weiterentwicklung der Gas-Austausch-Einheit, ist vorgesehen, dass sich die Querschnittsfläche des Einlasses asymmetrisch aufweitet. Die Aufweitung kann asymmetrisch erfolgen, um die Strömung gezielt zu lenken und somit homogen zu verteilen.

In einer Weiterentwicklung der Gas-Austausch-Einheit, ist vorgesehen, dass der Einlass eine Querschnittsfläche aufweist, die über die Länge des Einlasses variabel ist.

In einer Weiterentwicklung der Gas-Austausch-Einheit, ist vorgesehen, dass die Gas-Austausch-Einheit einen Auslass aufweist, dessen Querschnittsfläche sich in Strömungsrichtung verkleinert. Das Fluid, insbesondere Blut, wird durch eine kontinuierliche Querschnittsflächenabnahme beschleunigt. Ein Verhältnis zwischen Durchmessererweiterung (dD) und Lauflänge (dL) kleiner als 1 ist vorteilhaft: dD/dL < 1 oder dD/2dL.

Gemäß einer anderen Weiterentwicklung der Gas-Austausch-Einheit, ist vorgesehen, dass die Gas-Austausch-Einheit einen Auslass aufweist, der eine Umlenkung umfasst. Um eine möglichst kompakte Gas-Austausch-Einheit auszubilden wird auf der Auslassseite die Strömung des Fluids, insbesondere Blut, um einen Winkel umgeleitet. Der Umlenkwinkel beträgt etwa 90° oder zwischen 70° bis 90°. Bei derartigen Umlenkungen kann es zu Sekundärwirbeln oder Strömungsablösungen des Fluids, insbesondere des Bluts, kommen. Durch eine kontinuierliche Abnahme der Querschnittsfläche des Auslasses wird das Fluid jedoch beschleunigt, so dass der Bildung von Sekundärwirbeln oder Strömungsablösungen entgegengewirkt wird oder diese nahezu entfallen.

Gemäß einem unabhängigen Erfindungsgedanken umfasst die Gas-Austausch-Einheit bzw. der Oxygenator eine, insbesondere im Wesentlichen mittig angeordnete, Entlüftungsvorrichtung. Sollte es zum Ansaugen von Luft kommen, sammelt sich diese aufgrund der Einlassgeometrie, gebildet aus Einlass und Einlassgehäuse, in der Mitte der rotierenden Strömung, insbesondere in einer Kavität der Gas-Austausch-Einheit. Dort wird die Luft durch die Entlüftungsvorrichtung abgesaugt. Die Entlüftungsvorrichtung ist auch ein Auslass für Luftbläschen, die sich im Betrieb an diesen Stellen ansammeln können. Während der Patientenbehandlung können die Entlüftungsvorrichtungen auch zur Blutentnahme genutzt werden.
Um die Bildung von Blutkoageln (Tromben) zu verhindern, müssen alle Bereiche der Entlüftungsvorrichtung gespült werden können oder es muss verhindert werden, dass Blut verweilt.

Die Entlüftungsvorrichtung kann derart ausgebildet sein, dass sie wahlweise in einen ersten Betriebszustand, bei dem ein Spülen der Entlüftungsvorrichtung realisiert werden kann oder in einen zweiten Betriebszustand überführt werden kann, bei dem ein Entlüften einer Kavität der Gas-Austausch-Einheit realisiert werden kann. Mittels der Entlüftungsvorrichtung lassen sich somit in vorteilhafterweise zwei technische Funktionen realisieren, nämlich das Entlüften aus der Kavität und das Spülen und somit Reinigen der Entlüftungsvorrichtung. Vorzugsweise wird die Entlüftungsvorrichtung nach einem Entlüftungsvorgang gespült. Somit kann verhindert werden, dass bei einem Entlüftungsvorgang gegebenenfalls mit herausgeströmte Blutreste in der Entlüftungsvorrichtung verbleiben. Alternativ kann die Entlüftungsvorrichtung wahlweise in den ersten Betriebszustand überführt werden, bei dem kein Entlüften der Kavität der Gas-Austausch-Einheit erfolgt, oder in den zweiten Betriebszustand überführt werden, bei dem ein Entlüften der Kavität der Gas-Austausch-Einheit erfolgt. Bei dieser Ausführung lässt sich mit der Entlüftungsvorrichtung nur eine technische Funktion, nämlich das Entlüften der Kavität realisieren.

Die Gas-Austausch-Einheit kann eine Unterdruckquelle aufweisen, die mit der Entlüftungsvorrichtung fluidisch verbunden werden kann. Durch Anlegen eines Unterdrucks in der Entlüftungsvorrichtung mittels der Unterdruckquelle kann auf einfache Weise sichergestellt werden, dass die in der Kavität befindliche Luft aus dieser abgeführt wird.

In einer Weiterentwicklung der Gas-Austausch-Einheit, ist vorgesehen, dass die Entlüftungsvorrichtung eine flexible Membran umfasst. Der mittlere Bereich des Einlasses ist aus einem flexiblen Material gefertigt, so dass dieser sich zu Entlüftungszwecken ausstülpen lässt, so dass sich ein größeres Volumen auf der Blutseite ergibt, um die Luftblasen zu sammeln.

Die Gas-Austausch-Einheit kann ein Verstellelement aufweisen, das zum Überführen der Entlüftungsvorrichtung in den ersten Betriebszustand oder in den zweiten Betriebszustand dient. Das Verstellelement kann, wie nachstehend näher ausgeführt ist, je nach Einsatzfall unterschiedlich ausgebildet sein.

Die Entlüftungsvorrichtung kann einen Verschließkolben aufweisen, der zum Realisieren des ersten Betriebszustands in eine erste Stellung oder zum Realisieren des zweiten Betriebszustands in eine zweite Stellung überführt wird. Dabei kann der Verschließkolben linear beweglich und/oder drehbar gelagert sein, damit dieser von der ersten Stellung in die zweite Stellung oder umgekehrt überführt werden kann. Insbesondere kann der Verschließkolben um seine Längsachse drehbar gelagert sein. Alternativ oder zusätzlich kann der Verschließkolben derart ausgebildet sein, dass zum Überführen des Verschließkolbens von der ersten Stellung in die zweite Stellung ein Kolbenabschnitt relativ zu einem anderen Kolbenabschnitt bewegt wird.

Der Verschließkolben kann eine Fluidleitung aufweisen, die in der ersten Stellung des Verschließkolbens mit der Kavität fluidisch nicht verbunden ist und in der zweiten Stellung des Verschließkolbens mit der Kavität fluidisch verbunden ist. Darüber hinaus kann die Fluidleitung in der ersten Stellung des Verschließkolbens mit einer Zuführleitung zum Zuführen eines Spülmittels fluidisch verbunden und in der zweiten Stellung des Verschließkolbens nicht mit der Zuführleitung fluidisch verbunden sein. In der zweiten Stellung kann die in der Kavität befindliche Luft über die Fluidleitung aus der Kavität abgeführt werden. Bei dieser Ausführung kann der Verschließkolben drehbar gelagert sein und das Verstellelement kann derart ausgeführt sein, dass bei einem Betätigen des Verstellelements der Verschließkolben gedreht wird. Vorzugsweise kann das Verstellelement mit dem Verschließkolben drehfest verbunden sein.

Bei einer Weiterentwicklung kann ein Dichtring der Entlüftungsvorrichtung am Verschließkolben angebracht sein. Darüber hinaus kann die Entlüftungsvorrichtung ein Rückstellelement aufweisen, das mit dem Verschließkolben derart wirkverbunden ist, dass das Rückstellelement den Verschließkolben aus der zweiten Stellung in die erste Stellung drückt. Der Verschließkolben kann bei dieser Ausführung linear beweglich gelagert sein. Das Rückstellelement kann eine Feder, insbesondere eine Druckfeder, sein. Zum Überführen des Verschließkolbens aus der ersten Stellung in die zweite Stellung wird mittels des Verstellelements eine Kraft, insbesondere eine Linearkraft, auf den Verschließkolben ausgeübt. Das Verstellelement kann beispielsweise eine Spritze sein.

Der Verschließkolben kann bei einer Weiterentwicklung eine Schwächung aufweisen, die derart ausgebildet ist, dass bei der zweiten Stellung das Verstellelement durch den Verschließkolben hindurch tritt. Bei der ersten Stellung des Verschließkolbens kann der Verschließkolben trotz Schwächung einen Austritt von Luft oder Blut aus der Kavität verhindern. Das Verstellelement kann eine Pippette sein, die an ihrem durch den Verschließkolben hindurch tretenden Abschnitt wenigstens eine Öffnung aufweist. Dabei kann die in der Kavität befindliche Luft über die Öffnung und das Verstellelement aus der Kavität abgeführt werden. Die Schwächung des Verschließkolbens kann durch einen Schnitt im Verschließkolben realisiert werden.

Um die Gas-Austausch-Einheit weiter zu verbessern ist in einer Weiterentwicklung vorgesehen, dass die Hohlfasermatten in der Gas-Austausch-Einheit eingebettet sind und ein Übergang von den eingebetteten Hohlfasermatten zu angrenzenden Bauteilen schräge Übergänge aufweist. Die Position des Übergangs von den freien Fasern zu den eingebetteten Fasern (Verpottungsspiegel) kann ungenauen Toleranzen unterliegen. Durch die schrägen Übergänge mit denen die angrenzenden blutführenden Bauteile versehen sind, ergibt sich auch bei unterschiedlichen Positionen des Verpottungsspiegels ein glatter, stoßfreier und somit blutfreundlicher Übergang. Mit der Maßnahme der schrägen Übergänge wird auch das Herstellungsverfahren der Gas-Austausch-Einheit stark vereinfacht, da bei der Produktion die Toleranzen in einem breiteren Bereich nicht zu Qualitätseinbußen führen. In einer Weiterentwicklung der Gas-Austausch-Einheit, ist vorgesehen, dass das Einlassgehäuse Stabilisatoren aufweist. Um eine Stabilitätserhöhung des Einlassgehäuses bei der Verpottung zu erzielen, weist das Einlassgehäuse Stabilisatoren auf. Diese können als Stege oder Querstreben ausgebildet sein. Bei einer Verpottung, bei der hohe Temperaturen und/oder Spannungen erreicht werden können, bleibt so die Einlassgeometrie erhalten und ändert sich im Wesentlichen nicht durch Temperaturänderungen.

Üblicherweise werden bei bekannten Oxygenatoren die Hohlfasern an den Enden zunächst verschlossen und dann mit einem Polyurethanklebstoff eingebettet. Dieser Pottingschritt wird im Schleuderverfahren durchgeführt, um ein Verkleben der Fasern im späteren blutführenden Bereich durch Kapillareffekte zu verhindern und einen definierten Übergang zwischen der Verpottungsmasse und den freien Fasern zu ermöglichen. Anschließend werden die Fasern mit der ausgehärteten Verpottungsmasse von außen quer zur Faserrichtung aufgeschnitten um somit später ein Durchströmen der Fasern mit dem Gas zu ermöglichen. Üblicherweise erfolgt der Verpottungsschritt an beiden Enden der Fasern und somit ergeben sich zwei Verpottungsvorgänge bei Oxygenatoren mit parallel angeordneten Fasern oder vier Verpottungsvorgänge bei gestapelten Fasermatten.

Erfindungsgemäß wird ein Verfahren zum Herstellen einer Gas-Austausch-Einheit angegeben. Das Verfahren umfasst: Einbringen der Vergussmasse zum Einbetten der Faserenden, wobei das Einbringen einmalig erfolgt, und Ausbilden einer zylindrischen Kavität im zentralen Bereich der Gas-Austausch-Einheit.

Die erfindungsgemäße Gas-Austausch-Einheit wird aus gestapelten Hohlfasermatten hergestellt. Beim Verfahren zum Herstellen einer Gas-Austausch-Einheit wird die Vergussmasse zum Einbetten der Faserenden in einem Schritt in einer Zentrifuge eingebracht, so dass sich im zentralen Bereich der Gas-Austausch-Einheit eine zylindrische Kavität ergibt, in der die Hohlfasern mit einem Fluid, insbesondere Blut, in Kontakt kommen. Durch die zylindrische Kavität ergibt sich eine homogene Durchströmung der Fasermatten. Voraussetzung dafür ist eine gleichmäßige stirnseitige Anströmung der zylindrischen Kavität. Durch die gewählte Bauform ist eine Gas-Austausch-Einheit verwirklicht mit der die Produktionskosten durch Reduktion der Fertigungsschritte gering sind, da das Einbetten der Fasern in einem Arbeitsschritt erfolgt. Bei marktüblichen Gas-Austausch-Einheiten erfolgt das Einbetten der Fasern in zwei oder gar vier zeitintensiven Schritten in einer Zentrifuge.

Zudem werden mit dem beschriebenen Verfahren alle Bauteile, die mit dem Blut in Kontakt kommen, in einem Arbeitsschritt gefügt. Es sind keine weiteren Verklebungen notwendig.

Im Gegensatz dazu wird bei den bekannten Oxygenatoren die homogene Durchströmung der Fasermatten durch Verteiler- oder Diffusorplatten gemacht, die gezielt den Strömungswiderstand unterschiedlich stark erhöhen und somit den Blutfluss verteilen. Von Nachteil ist dabei, dass so zusätzliche Scherbelastung und Irritation das Blut schädigen kann. Auch können auf den Rückseitigen strömungsabgeschatteten Bereichen der Verteilerplatten Thromben entstehen, da dort Totwassergebiete entstehen können. Mit den Maßnahmen der Erfindung kann auf solche Platten verzichtet werden.

### KURZE BESCHREIBUNG DER FIGUREN

Weitere Einzelheiten der Erfindung sind den Ausführungsbeispielen zu entnehmen, die im Folgenden anhand der Figuren beschrieben wird. Es zeigen:
Figur 1: ein Ausführungsbeispiel einer Gas-Austausch-Einheit,
Figur 2: ein weiteres Ausführungsbeispiel einer Gas-Austausch-Einheit,
Figur 3: das Einlassgehäuse aus Figur 2,
Figur 4: ein Ausführungsbeispiel einer Oberfläche eines Einlassgehäuses,
Figur 5: eine Querschnittsansicht eines Einlassgehäuses,
Figur 6: ein Ausführungsbeispiel einer Entlüftungsvorrichtung gemäß einer ersten Ausführung,
Figur 7: eine perspektivische Darstellung einer Entlüftungsvorrichtung gemäß einer zweiten Ausführung,
Figur 8: eine vergrößerte Darstellung eines in Figur 7 dargestellten Abschnitts, wobei sich die Entlüftungsvorrichtung in einem ersten Betriebszustand befindet,
Figur 9: eine Schnittdarstellung des in Figur 8 gezeigten Abschnitts,
Figur 10: eine vergrößerte Darstellung eines in Figur 7 dargestellten Abschnitts, wobei sich die Entlüftungsvorrichtung in einem zweiten Betriebszustand befindet,
Figur 11: eine Schnittdarstellung des in Figur 10 gezeigten Abschnitts,
Figur 12: eine perspektivische Darstellung einer Entlüftungsvorrichtung gemäß einer dritten Ausführung,
Figur 13: eine vergrößerte Darstellung eines in Figur 12 dargestellten Abschnitts, wobei sich die Entlüftungsvorrichtung in einem ersten Betriebszustand befindet,
Figur 14: eine Schnittdarstellung des in Figur 13 gezeigten Abschnitts,
Figur 15: eine vergrößerte Darstellung eines in Figur 12 dargestellten Abschnitts, wobei sich die Entlüftungsvorrichtung in einem zweiten Betriebszustand befindet,
Figur 16: eine Schnittdarstellung des in Figur 15 gezeigten Abschnitts,
Figur 17: eine Schnittdarstellung einer Entlüftungsvorrichtung gemäß einer vierten Ausführung, bei der sich die Entlüftungsvorrichtung in einem ersten Betriebszustand befindet,
Figur 18: eine Schnittdarstellung der Entlüftungsvorrichtung gemäß der vierten Ausführung, bei der sich die Entlüftungsvorrichtung in einem zweiten Betriebszustand befindet,
Figur 19: eine Schnittdarstellung einer Entlüftungsvorrichtung gemäß einer fünften Ausführung, bei der sich die Entlüftungsvorrichtung in einem ersten Betriebszustand befindet,
Figur 20: eine Schnittdarstellung der Entlüftungsvorrichtung gemäß der fünften Ausführung, bei der sich die Entlüftungsvorrichtung in einem zweiten Betriebszustand befindet,
Figur 21: eine Schnittdarstellung einer Entlüftungsvorrichtung gemäß einer sechsten Ausführung, bei der sich die Entlüftungsvorrichtung in einem ersten Betriebszustand befindet,
Figur 22: eine Schnittdarstellung der Entlüftungsvorrichtung gemäß der sechsten Ausführung, bei der sich die Entlüftungsvorrichtung in einem zweiten Betriebszustand befindet,
Figur 23: ein Ausführungsbeispiel eines Auslassgehäuses,
Figur 24: eine Querschnittsansicht eines Auslasses.

### AUSFÜHRLICHE BESCHREIBUNG DER FIGUREN

Im Folgenden werden exemplarische Ausführungsformen der Erfindung unter Bezugnahme auf die beiliegenden Zeichnungen beschrieben:

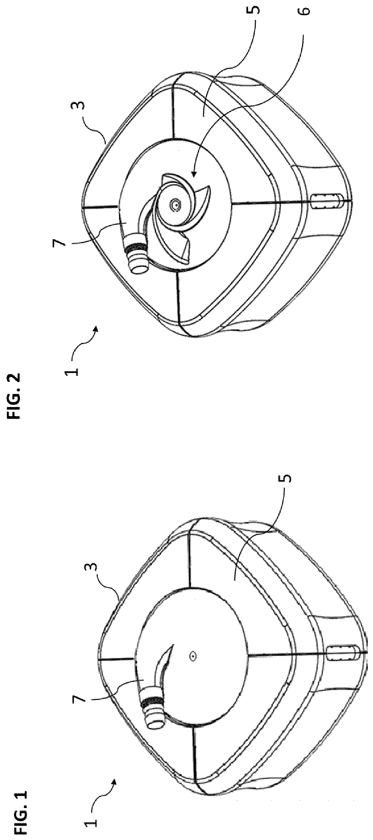

Figur 1 zeigt ein Ausführungsbeispiel einer Gas-Austausch-Einheit 1, welche Hohlfasermatten (nicht gezeigt) umfasst. Die Gas-Austausch-Einheit 1 umfasst einen Einlass 7, der azentrisch auf einem Einlassgehäuse 5 angeordnet ist. Der Einlass 7 ist zum Einlassgehäuse 5 hin schräggestellt. Der Einlass 7 ist stirnseitig an einem Gehäuse der Gas-Austausch-Einheit 1, das das Einlassgehäuse 5 aufweist, angeordnet und ermöglicht eine homogene Durchströmung der Hohlfasermatten. Das Einlassgehäuse 5 bildet eine Stirnseite des Gehäuses der Gas-Austausch-Einheit 1 aus. Das Einlassgehäuse 5 wird in seinem Randbereich von einem Gehäuseteil 3 der Gas-Austausch-Einheit 1 überdeckt (vgl. z.B. Figur 1).

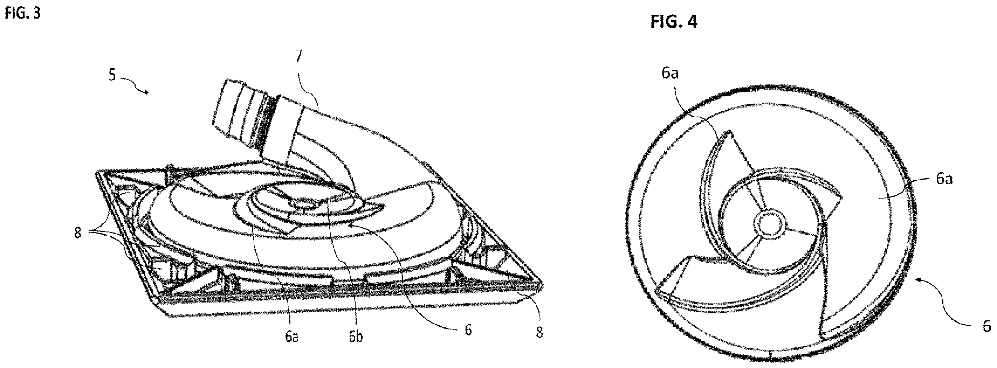

Figur 2 zeigt ein weiteres Ausführungsbeispiel einer Gas-Austausch-Einheit 1. Die Oberfläche des Einlassgehäuses 5 weist Strukturen 6 auf, die auf der inneren blutführenden Oberfläche des Einlassgehäuses 5 die Strömung des durchfließenden Blutes zusätzlich möglichst homogen verteilen. Das Einlassgehäuse 5 kann optional in Figur 3 gezeigte Stabilisatoren 8 aufweisen. Die Stabilisatoren 8 können als Stege oder Querstreben ausgebildet sein. Bei einer Verpottung, bei der hohe Temperaturen und/oder Spannungen erreicht werden können, bleibt so die Einlassgeometrie des Einlassgehäuses 5 erhalten und ändert sich im Wesentlichen nicht durch Temperaturänderungen und/oder Spannungen.

Figur 3 zeigt das Einlassgehäuse 5 aus Figur 2. Die azentrische schräge Anordnung des Einlasses 7 ist deutlich erkennbar. Die Strukturen 6, welche die Strömung homogen in der Gas-Austausch-Einheit 1 verteilen sind als schaufelförmige Strukturen 6a und als stegförmige Strukturen 6b ausgebildet. Die Strukturen 6 können auch als Querstreben auf der Oberfläche des Einlassgehäuses 5 angeordnet sein.

Durch diese Strukturen 6 kann die Durchströmung bzw. Beschickung der Faserflächen der Hohlfasermatten in der Gas-Austausch-Einheit 1 gleichmäßig im inneren, mittigen Bereich der Hohlfasermatten als auch im äußeren Bereich, der einen Randbereich der Hohlfasermatten umfasst, erfolgen.

Figur 4 zeigt ein Ausführungsbeispiel einer strukturierten Oberfläche eines Einlassgehäuses 5 in Draufsicht. Die unterschiedliche Größe der schaufelförmigen Strukturen 6a ist erkennbar. Dadurch wird eine gleichmäßige, homogene Durchströmung der Gas-Austausch-Einheit 1 gewährleistet.

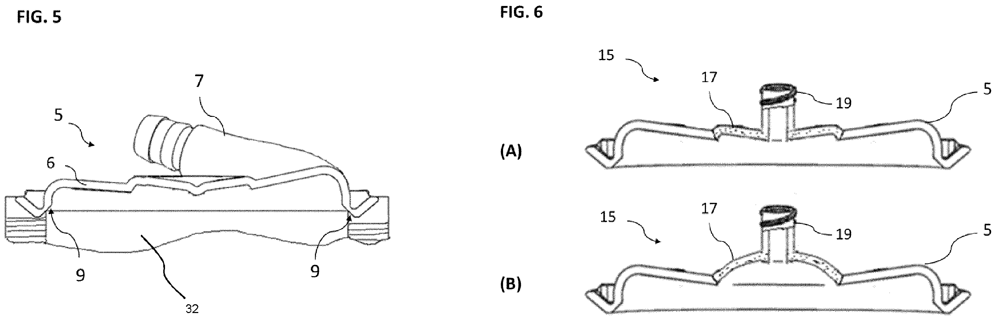

Figur 5 zeigt eine Querschnittsansicht eines Einlassgehäuses 5. Auch in dieser Figur ist die Schrägstellung des Einlasses 7, als auch die azentrische Anordnung ersichtlich. Das Einlassgehäuse 5 weist die Strukturen 6 auf. Die Hohlfasermatten 32 sind in der Gas-Austausch-Einheit 1 eingebettet und mit dem Einlassgehäuse 5 in Kontakt. Im Kontaktbereich der Hohlfasermatten 32 mit dem Einlassgehäuse 5 weist das Einlassgehäuse 5 schräge Übergänge 9 auf. Die Position des jeweiligen Übergangs 9 von den freien Fasern zu den eingebetteten 10 Fasern (Verpottungsspiegel) kann ungenauen Toleranzen unterliegen.

Durch die schrägen Übergänge 9 ergibt sich auch bei unterschiedlichen Positionen des Verpottungsspiegels ein glatter, stoßfreier und somit blutfreundlicher Übergang.

Figur 6 zeigt ein Ausführungsbeispiel einer Entlüftungsvorrichtung 15. Sollte es zum Ansaugen von Luft kommen, sammelt sich diese aufgrund der Einlassgeometrie, gebildet aus Einlass 7 und Einlassgehäuse 5, in der Mitte der rotierenden Strömung. Dort wird die Luft durch die Entlüftungsvorrichtung 15 abgesaugt. Die Entlüftungsvorrichtung 15 ist auch ein Auslass für Luftbläschen. Die Entlüftungsvorrichtung 15 umfasst ein Entlüftungsrohr 19.

Die Entlüftungsvorrichtung 15 umfasst eine flexible Membran 17. Teil (A) der Figur 6 zeigt die Membran 17 in einem Zustand ohne Entlüftung. Bei einer Entlüftung, in Teil (B) der Figru 7 gezeigt, wird die Membran 17 ausgestülpt, so dass sich ein vergrößertes Volumen auf der Blutseite ergibt, um die Luftblasen zu sammeln.

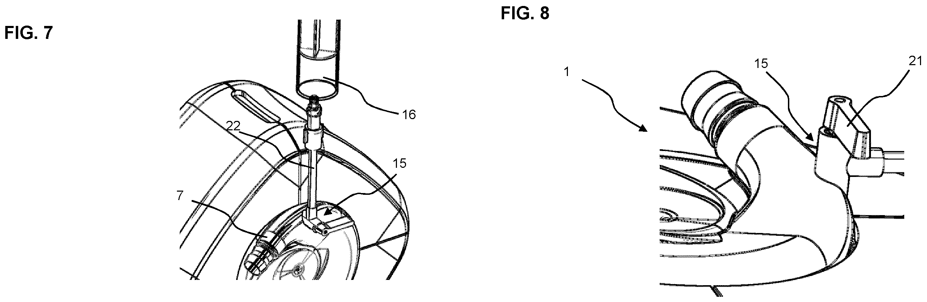

Figur 7 zeigt eine perspektivische Darstellung einer Entlüftungsvorrichtung 15 gemäß einer zweiten Ausführung. Die Entlüftungsvorrichtung 15 ist benachbart zu dem Einlass 7 angeordnet. Die Entlüftungsvorrichtung 15 ist außerdem mittels einer Leitung 22 mit einer Unterdruckquelle 16 fluidisch verbunden. Die Unterdruckquelle 16 ist als Spritze ausgeführt.

Figur 8 zeigt eine vergrößerte Darstellung eines in Figur 7 gezeigten Abschnitts. Die Gas-Austausch-Einheit 1 weist ein Verstellelement 21 in Form eines Drehhebels auf, mittels dem die Entlüftungsvorrichtung 15 wahlweise in einen ersten Betriebszustand oder in einen zweiten Betriebszustand überführt werden kann. Bei der in Figur 8 gezeigten Ausführung befindet sich die Entlüftungsvorrichtung in dem ersten Betriebszustand.

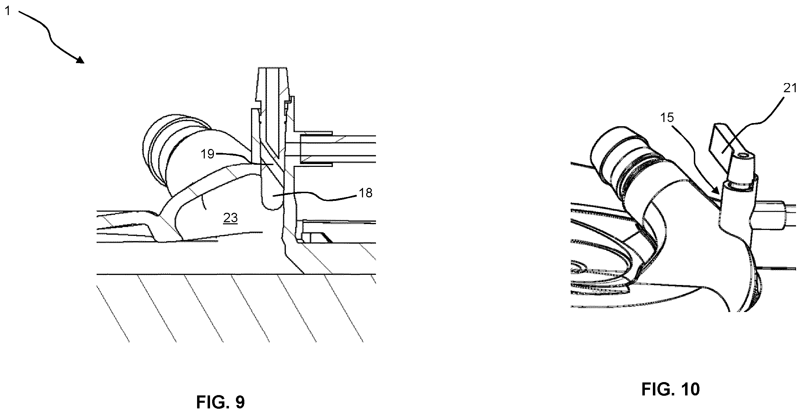

Figur 9 zeigt eine Schnittdarstellung des in Figur 8 dargestellten Abschnitts. Wie aus Figur 9 ersichtlich ist, weist die Entlüftungsvorrichtung 15 einen Verschließkolben 18 auf, der mit dem Verstellelement 21 drehfest verbunden ist. In dem Verschließkolben 18 ist eine Fluidleitung 19 vorhanden. Bei einer in Figur 8 dargestellten ersten Stellung des Verschließkolbens 18 ist die Fluidleitung 19 so orientiert, dass die Fluidleitung 19 mit der Leitung 22 fluidisch nicht verbunden ist. Bei dieser Ausführung ist ein Spülen der Entlüftungsvorrichtung nicht möglich.

Figur 10 zeigt ebenfalls eine vergrößerte Darstellung eines in Figur 7 gezeigten Abschnitts. Bei der in Figur 10 dargestellten Ausführung wurde das Verstellelement 21 gedreht, so dass sich die Entlüftungsvorrichtung 15 in dem zweiten Betriebszustand befindet.

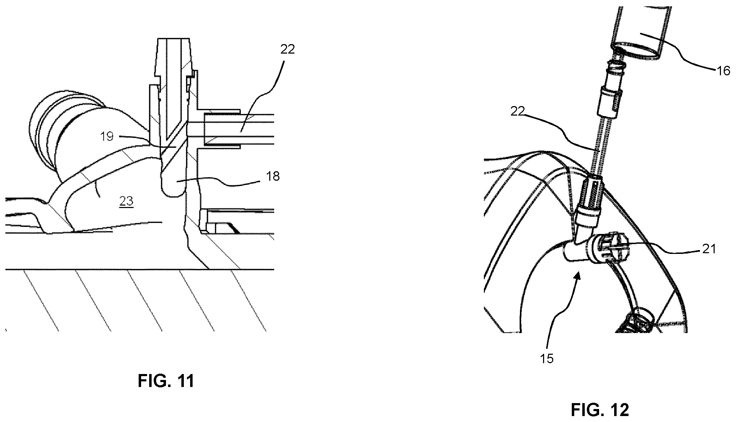

Figur 11 zeigt eine Schnittdarstellung des in Figur 10 gezeigten Abschnitts. Bei dem zweiten Betriebszustand der Entlüftungsvorrichtung 15 ist der Verschließkolbens 18 in einer zweiten Stellung angeordnet, bei der die Fluidleitung 19 mit der Kavität 23 der Gas-Austausch-Einheit 1 fluidisch verbunden ist. Darüber hinaus ist die Kavität 23 mit der Leitung 22 und somit mit der in Figur 7 dargestellten Unterdruckquelle 16 fluidisch verbunden. Bei der zweiten Stellung des Verschließkolbens 18 kann die in der Kavität 23 angesammelte Luft über die Fluidleitung 19 und die Leitung 20 abgeführt werden.

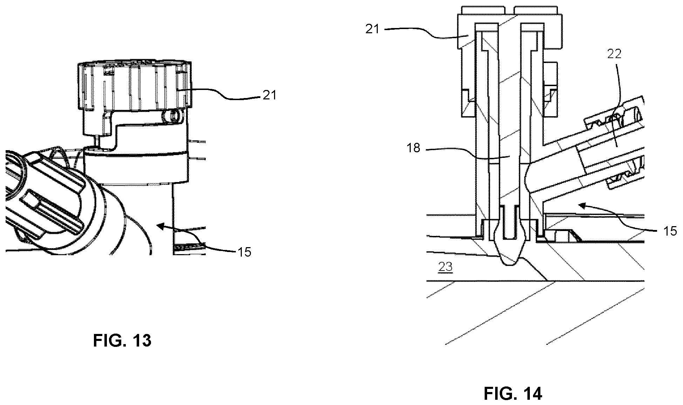

Figur 12 zeigt eine perspektivische Darstellung einer Entlüftungsvorrichtung gemäß einer dritten Ausführung. Die Entlüftungsvorrichtung 15 ist über die Leitung 22 mit der Unterdruckquelle 16 fluidisch verbunden. Dabei unterscheidet sich die in Figur 12 dargestellte Ausführung von der in Figur 7 dargestellten Ausführung in der Ausbildung des Verstellelements 21. Aus Figur 13 ist ersichtlich, dass dass Verstellelement 21 als Drehknopf ausgeführt ist.

Figur 14 zeigt eine Schnittdarstellung der in Figur 13 gezeigten Entlüftungsvorrichtung 15 und des Verstellelements 21. Wie aus Figur 14 ersichtlich ist, besteht in der ersten Stellung des Verschließkolbens 18 keine fluidische Verbindung zwischen der Leitung 22 und der Kavität 23. Somit kann bei der ersten Stellung des Verschließkolbens 18 die in der Kavität 23 befindliche Luft nicht abgeführt werden. Der Verschließkolben 18 ist mit dem Verstellelement 21 drehfest verbunden.

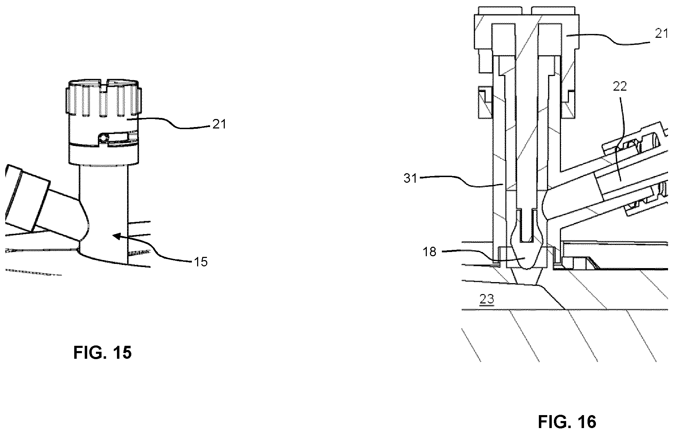

Figur 15 zeigt eine vergrößerte Darstellung der in Figur 12 gezeigten Entlüftungsvorrichtung 15 und des Verstellelements 21, wobei sich die Entlüftungsvorrichtung 15 in dem zweiten Betriebszustand befindet. Zum Überführen der Entlüftungsvorrichtung 15 aus dem ersten Betriebszustand in den zweiten Betriebszutand wird das Verstellelement 21 gedreht.

Figur 16 zeigt eine Schnittdarstellung der in Figur 15 gezeigten Entlüftungsvorrichtung 15 und des Verstellelements 21. Bei der zweiten Stellung des Verschließkolbens 18 besteht eine fluidische Verbindung zwischen der Kavität 23 und der Leitung 22. Zum Überführen des Verschließkolbens 18 von der ersten Stellung in die in Figur 16 gezeigte zweite Stellung wird der Verschließkolben 18 linear bewegt. Die in der Kavität 23 angesammelte Luft kann über einen Zwischenraum zwischen dem Verschließkolben 18 und einem Entlüftungsvorrichtungsgehäuse 31 und über die Leitung 22 zu der Unterdruckquelle 16 abgeführt werden.

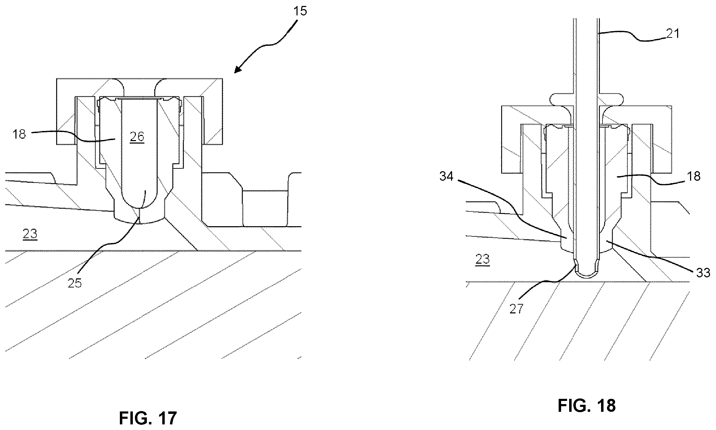

Figur 17 zeigt eine Schnittdarstellung der Entlüftungsvorrichtung gemäß einer vierten Ausführung. Der Verschließkolben 18 der Entlüftungsvorrichtung 15 unterscheidet sich von den oben beschriebenen Verschließkolben dadurch, dass er eine Schwächung 25 aufweist. Die Schwächung 25 entspricht einem Schnitt im Verschließkolben 18. Dabei ist die Schwächung 25 in einem Bereich des Verschließkolbens 18 angeordnet, der sich in die Kavität 23 erstreckt. Ein weiterer Unterschied besteht darin, dass der Verschließkolben 18 eine Aussparung 26 aufweist. In Figur 17 befindet sich der Verschließkolben 18 in der ersten Stellung, so dass die in der Kavität 23 angesammelte Luft nicht abgeführt werden kann.

Figur 18 zeigt eine Schnittdarstellung der Entlüftungsvorrichtung 15 gemäß der vierten Ausführung, wobei sich die Entlüftungsvorrichtung 15 in dem zweiten Betriebszustand befindet. Wie aus Figur 18 ersichtlich ist, dringt das Verstellelement 21 durch den Verschließkolben 18 hindurch. Insbesondere dringt das Verstellelement 21 in dem Bereich der Schwächung 25 des Verschließkolbens 18 durch diesen hindurch. Infolge des hindurch tretens des Verstellelements 21 durch den Verschließkolben 18 bewegen sich ein Kolbenabschnitt 33 und ein anderer Kolbenabschnitt 34 voneinander weg. Dabei ist das Verstellelement 21 teilweise in der Aussparung 26 angeordnet. Das Verstellelement 21 weist an seinem in der Kavität 23 liegenden Abschnitt eine Öffnung 27 auf, über die Luft aus der Kavität 23 abgeführt wird.

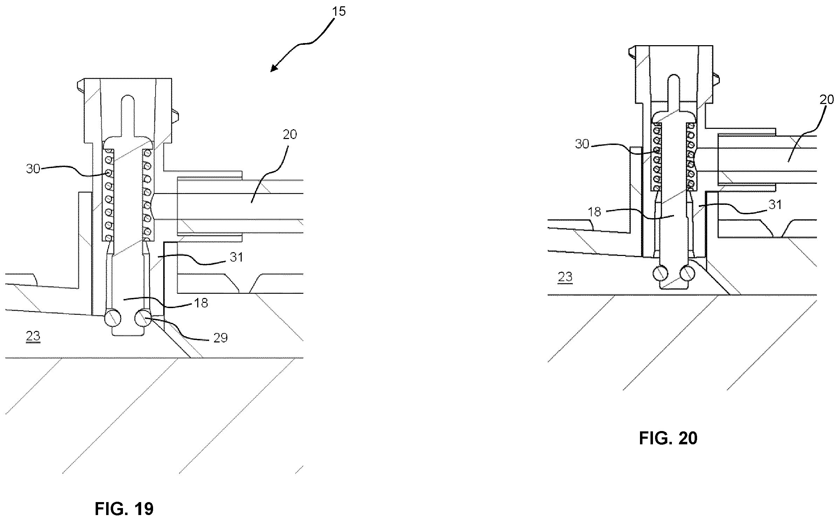

Figur 19 zeigt eine Schnittdarstellung der Entlüftungsvorrichtung 15 gemäß einer fünften Ausführung. Die Entlüftungsvorrichtung 15 weist einen Dichtring 29 auf, der an dem Verschließkolben 18 angebracht ist. Darüber hinaus weist die Entlüftungsvorrichtung 15 ein Rückstellmittel 30 auf, das derart ausgebildet ist, dass es den Verschließkolben 18 aus der in Figur 20 gezeigten zweiten Stellung in die in Figur 19 gezeigte erste Stellung drückt. Das Rückstellelement 30 stützt sich an einem Ende an dem Verschließkolben 18 und an dem anderen Ende an einem Entlüftungsvorrichtungsgehäuse 31 ab.

Figur 20 zeigt eine Schnittdarstellung der Entlüftungsvorrichtung 15 gemäß der fünften Ausführung, bei der sich die Entlüftungsvorrichtung 15 in dem zweiten Betriebszustand befindet. Wie aus Figur 20 ersichtlich ist, wird der Verschließkolben 18 durch ein nicht dargestelltes Verstellelement 21 linear bewegt. Insbesondere wird der Verschließkolben 18 soweit in die Kavität 23 reingedrückt, dass ein Zwischenraum zwischen dem Verschließkolben 18 und dem Entlüftungsvorrichtungsgehäuse besteht, durch den in der Kavität 23 angesammelte Luft abgeführt wird. Die aus der Kavität abgeführte Luft strömt über die Leitung 20 zu der Unterdruckquelle 16. Bei dem Überführen des Verschließkolbens 18 in die zweite Stellung wird das Rückstellelement 30 gespannt.

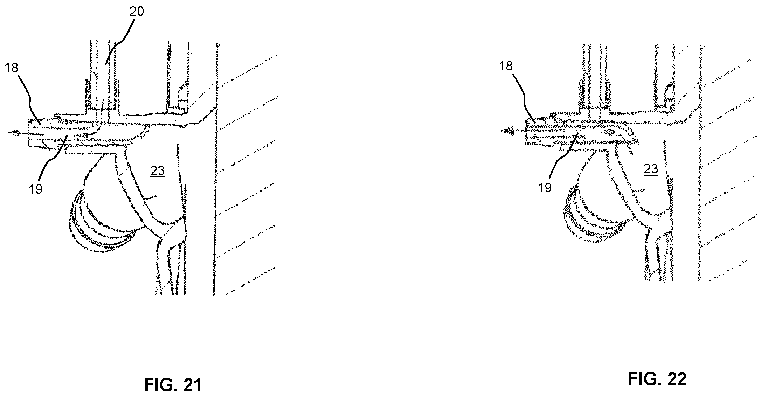

Figur 21 zeigt eine Schnittdarstellung einer Entlüftungsvorrichtung 15 gemäß einer sechsten Ausführung, bei der sich die Entlüftungsvorrichtung 15 in einem ersten Betriebszustand befindet, bei dem ein Spülen der Entlüftungsvorrichtung 15 erfolgt. Bei der in der Figur 21 gezeigten ersten Stellung des Verschließkolbens 18 ist die Fluidleitung 19 des Verschließkolbens 18 mit einer Zuführleitung 20 fluidisch verbunden. Über die Zuführleitung 20 wird ein Spülmittel zugeführt, das, wie durch die Pfeile gezeigt ist, durch die Fluidleitung 19 strömt und aus dem Verschließkolben 18 austritt. Bei dem ersten Betriebszustand besteht keine fluidische Verbindung zwischen der Fluidleitung 19 und der Kavität 23.

Figur 22 zeigt eine Schnittdarstellung einer Entlüftungsvorrichtung 15 gemäß einer sechsten Ausführung, bei der sich die Entlüftungsvorrichtung 15 in einem zweiten Betriebszustand befindet, bei dem der Entlüftungsvorgang erfolgt. Bei der zweiten Stellung des Verschließkolbens 18 besteht eine fluidische Verbindung zwischen der Fluidleitung 19 und der Kavität 23. Die in der Kavität 23 befindliche Luft kann, wie durch die Pfeile gezeigt ist, durch die Fluidleitung 19 abgeführt werden.

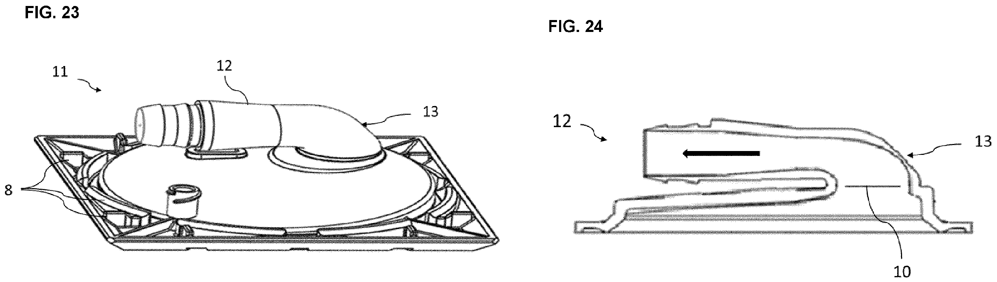

Figur 23 zeigt ein Ausführungsbeispiel eines Auslassgehäuses 11. Das Auslassgehäuse 11 weist einen Auslass 12 auf. Die Querschnittsfläche des Auslasses 12 verkleinert sich in Strömungsrichtung, wie in der Querschnittsansicht des Auslasses in Figur 22 gezeigt ist. Das Einlassgehäuse 5, das Auslassgehäuse 11 und der Gehäuseteil 3 bilden ein Gehäuse der Gas-Austausch-Einheit 1.

Der Auslass 12 weist eine Umlenkung 13 auf. Der Umlenkwinkel beträgt 90°, kann jedoch auch zwischen 70° bis 90° sein. Durch die kontinuierliche Abnahme der Querschnittsfläche des Auslasses 12 wird das Fluid (Blut) beschleunigt, so dass der Bildung von Sekundärwirbeln oder Strömungsablösungen wirksam entgegengewirkt wird.

Das Auslassgehäuse 11 weist optional Stabilisatoren 8 auf, die als Stege oder Querstreben ausgebildet sein können. Bei einer Verpottung, bei der hohe Temperaturen erreicht werden können, bleibt so die Geometrie des Auslassgehäuses 11 erhalten und ändert sich im Wesentlichen nicht durch Temperaturänderungen.

Figur 24 zeigt eine Querschnittsansicht des Auslasses 12. Die Querschnittsfläche 10 des Auslasses 12 verengt sich kontinuierlich in Strömungsrichtung des Fluids (Blut) im Bereich der Änderung der Hauptströmungsrichtung (Umlenkung 13). Die Strömungsrichtung ist durch einen Pfeil angedeutet. Die Verengung kann asymmetrisch erfolgen. Die Querschnittsfläche 10 kann über die Länge des Auslasses 12 variabel sein. Das Fluid (Blut) wird durch eine kontinuierliche Querschnittsflächenabnahme auf der Auslassseite der Gas-Austausch-Einheit 1 beschleunigt.

Es ist zu beachten, dass die in diesem Dokument beschriebenen Verfahren, Vorrichtungen und Systeme sowohl alleine, als auch in Kombination mit anderen in diesem Dokument beschriebenen Verfahren, Vorrichtungen und Systemen verwendet werden können. Des Weiteren können jegliche Aspekte der in diesem Dokument beschriebenen Verfahren, Vorrichtung und Systemen in vielfältiger Weise miteinander kombiniert werden. Insbesondere können die Merkmale der Ansprüche in vielfältiger Weise miteinander kombiniert werden.

Die Erfindung ist anhand der Zeichnungen und der obigen Beschreibung ausführlich beschrieben. Diese Erfindung kann jedoch in vielen verschiedenen Formen ausgeführt werden und sollte nicht als auf die hier dargelegten Ausführungsformen begrenzt ausgelegt werden; vielmehr sind diese Ausführungsformen vorgesehen, damit diese Offenbarung gründlich und vollständig ist, und decken den Schutzumfang der Erfindung für einen Fachmann vollständig ab. Die Terminologie, die in der ausführlichen Beschreibung der in den beiliegenden Zeichnungen dargestellten Ausführungsformen verwendet wird, soll für die Erfindung nicht einschränkend sein. In den Zeichnungen beziehen sich gleiche Zeichen auf gleiche Elemente.

## Patentansprüche

1. Gas-Austausch-Einheit umfassend Hohlfasermatten, umfassend einen Einlass, der azentrisch schräggestellt ist.

2. Gas-Austausch-Einheit nach Anspruch 1, wobei der Einlass stirnseitig an einem Gehäuse der Gas-Austausch-Einheit angeordnet ist.

3. Gas-Austausch-Einheit nach Anspruch 1 oder 2, wobei der Einlass mit einem Einlassgehäuse verbunden ist, dessen Oberfläche Strukturen aufweist, wobei insbesondere die Strukturen schaufelförmig, stegförmig oder als Querstreben auf der Oberfläche des Einlassgehäuses angeordnet sind.

4. Gas-Austausch-Einheit nach einem der vorhergehenden Ansprüche, wobei der Einlass eine Querschnittsfläche aufweist, die sich in Strömungsrichtung kontinuierlich und/oder asymmetrisch aufweitet und/oder die über die Länge des Einlasses variabel ist.

5. Gas-Austausch-Einheit nach einem der vorhergehenden Ansprüche, wobei eine Durchmessererweiterung des Einlasses über die Lauflänge des Einlasses kleiner als 45° ist.

6. Gas-Austausch-Einheit nach einem der vorhergehenden Ansprüche, wobei die Gas-Austausch-Einheit einen Auslass aufweist, dessen Querschnittsfläche sich in Strömungsrichtung kontinuierlich verkleinert und/oder der eine Umlenkung umfasst.

7. Gas-Austausch-Einheit, insbesondere nach einem der vorhergehenden Ansprüche, umfassend eine, insbesondere im Wesentlichen mittig angeordnete, Entlüftungsvorrichtung, wobei insbesondere
a. die Entlüftungsvorrichtung wahlweise in einen ersten Betriebszustand, bei dem ein Spülen der Entlüftungsvorrichtung realisierbar ist, oder in einen zweiten Betriebszustand überführbar ist, bei dem ein Entlüften einer Kavität der Gas-Austausch-Einheit realisierbar ist oder
b. die Entlüftungsvorrichtung wahlweise in einen ersten Betriebszustand, bei dem kein Entlüften einer Kavität der Gas-Austausch-Einheit realisierbar ist, oder in einen zweiten Betriebszustand überführbar ist, bei dem ein Entlüften der Kavität der Gas-Austausch-Einheit realisierbar ist.

8. Gas-Austausch-Einheit nach Anspruch 7, wobei die Entlüftungsvorrichtung eine flexible Membran umfasst.

9. Gas-Austausch-Einheit nach einem der Ansprüche 7 oder 8, **gekennzeichnet durch** ein Verstellelement zum Überführen der Entlüftungsvorrichtung in den ersten Betriebszustand oder in den zweiten Betriebszustand, wobei insbesondere die Entlüftungsvorrichtung einen Verschließkolben aufweist, der zum Realisieren des ersten Betriebszustands in eine erste Stellung oder zum Realisieren des zweiten Betriebszustands in eine zweite Stellung überführbar ist und/oder der Verschließkolben zum Überführen von der ersten Stellung in die zweite Stellung oder umgekehrt
a. linear beweglich gelagert ist und/oder
b. drehbar gelagert ist und/oder
c. derart ausgebildet ist, dass ein Kolbenabschnitt relativ zu einem anderen Kolbenabschnitt bewegbar ist.

10. Gas-Austausch-Einheit nach Anspruch 9, **dadurch gekennzeichnet, dass** der Verschließkolben eine Fluidleitung aufweist, die
a. in der ersten Stellung mit der Kavität fluidisch nicht verbunden ist und in einer zweiten Stellung mit der Kavität fluidisch verbunden ist und/oder die
b. in der ersten Stellung mit einer Zuführleitung zum Zuführen eines Spülmittels fluidisch verbunden ist und in der zweiten Stellung nicht mit der Zuführleitung fluidisch verbunden ist.

11. Gas-Austausch-Einheit nach Anspruch 9 oder 10, **dadurch gekennzeichnet, dass**
a. ein Dichtring am Verschließkolben angebracht ist und/oder dass
b. ein Rückstellelement mit dem Verschließkolben derart wirkverbunden ist, dass das Rückstellelement den Verschließkolben aus der zweiten Stellung in die erste Stellung drückt.

12. Gas-Austauscheinheit nach einem der Ansprüche 9 bis 11, **dadurch gekennzeichnet, dass** der Verschließkolben eine Schwächung aufweist, die derart ausgebildet ist, dass
a. bei der zweiten Stellung des Verschließkolbens das Verstellelement durch den Verschließkolben hindurch tritt und/oder dass
b. bei der ersten Stellung des Verschließkolbens kein Entlüften realisierbar ist.

13. Gas-Austausch-Einheit nach einem der Ansprüche 1 bis 12, wobei die Hohlfasermatten in der Gas-Austausch-Einheit eingebettet sind und ein Übergang von den eingebetteten Hohlfasermatten zu angrenzenden Bauteilen schräge Übergänge aufweist.

14. Gas-Austausch-Einheit nach einem der vorhergehenden Ansprüche 3 bis 13, **dadurch gekennzeichnet, dass** das Einlassgehäuse Stabilisatoren aufweist.

15. Verfahren zum Herstellen einer Gas-Austausch-Einheit nach einem der Ansprüche 1 bis 14, umfassend
Einbringen der Vergussmasse zum Einbetten der Faserenden, wobei das Einbringen einmalig erfolgt,
Ausbilden einer zylindrischen Kavität im zentralen Bereich der Gas-Austausch-Einheit.
